(19) Europäisches Patentamt / European Patent Office / Office européen des brevets

(11) **EP 2 017 001 A1**

(12) **EUROPEAN PATENT APPLICATION**
published in accordance with Art. 153(4) EPC

(43) Date of publication:
**21.01.2009 Bulletin 2009/04**

(21) Application number: **07743008.0**

(22) Date of filing: **09.05.2007**

(51) Int Cl.:
**B01J 19/00** (2006.01)    **B01D 9/02** (2006.01)
**C07C 7/14** (2006.01)    **C07C 13/615** (2006.01)
**B01D 21/26** (2006.01)    **B04B 1/20** (2006.01)

(86) International application number:
**PCT/JP2007/059573**

(87) International publication number:
**WO 2007/129724 (15.11.2007 Gazette 2007/46)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **10.05.2006 JP 2006131274**

(71) Applicant: **Idemitsu Kosan Co., Ltd.**
**Chiyoda-ku**
**Tokyo 100-8321 (JP)**

(72) Inventor: **HOSOTANI, Noriaki**
**Ichihara-shi, Chiba 299-0193 (JP)**

(74) Representative: **Harmsen, Dirk**
**Vossius & Partner**
**Siebertstrasse 4**
**81675 München (DE)**

(54) **METHOD FOR TREATMENT OF CRYSTAL SLURRY**

(57)    The present invention provides a method for the treatment of a crystal slurry by centrifugal washing, including a centrifugal separation step in which a slurry obtained by crystallizing a desired substance from a solution containing an organic component is subjected to solid liquid separation. The centrifugal separation step includes carrying out centrifugal separation to form a cake, and then washing the formed cake with an organic solvent so that the residual organic solvent content of the obtained crystals can be reduced to 1% by mass or less.

EP 2 017 001 A1

## Description

Technical Field

The present invention relates to a method for the treatment of a crystal slurry by centrifugal washing, including a centrifugal separation step in which a slurry obtained by crystallizing a desired substance from a solution containing an organic component is subjected to solid liquid separation, the centrifugal separation step comprising carrying out centrifugal separation to form a cake, and then washing the formed cake with an organic solvent.

More specifically, the present invention is related to a method for the treatment of a crystal slurry by centrifugal washing, including a centrifugal separation step in which a slurry obtained by crystallizing a desired substance from a solution containing an organic component is solid-liquid separated into crystals and a mother liquor, wherein the mother liquor deposited onto and contained inside the crystals is efficiently washed off and separated using an organic solvent so that the residual organic solvent content of the obtained crystals can be reduced to 1% by mass or less.

Background Art

In a case where a desired substance contained in a liquid containing organic components is recovered by crystallizing the desired substance, a method is adopted which includes separating a slurry of the crystals into crystals and a mother liquor using a drum filter, a centrifugal separation machine, a horizontal belt filter or the like, and drying the obtained crystals in a drying step.

Patent Document 1 discloses a method for producing high purity adamantanes which comprises crystallizing adamantanes obtained by isomerizing trimethylenenorbornane by a crystallization method, subjecting the obtained crystallization liquid to solid-liquid separation by an ordinary method, such as vacuum filtration or centrifugation, using a filter cloth or a sintered metal, and then washing the obtained crude adamantanes with a washing solvent.

Patent Document 2 discloses a cleaning method using a filtration machine adapted to separate a crystal-containing liquid into a crystal component and a mother liquor with a filter medium, the cleaning method including filling a cleaning liquid in a rear face part of the filter medium to dissolve and recover crystals accumulating and adhering on the rear face of the filter medium.

However, no method for the treatment of a crystal slurry by centrifugal washing is known which includes a centrifugal separation step in which a mother liquor deposited onto and contained inside the crystals is efficiently washed off and separated using an organic solvent so that the residual organic solvent content of the obtained crystals can be reduced to 1% by mass or less and, therefore, substantially no drying step is needed.

[Patent Document 1] Japanese Unexamined Patent Application Publication No. 2004-59510
[Patent Document 2] Japanese Unexamined Patent Application Publication No. H07-47209

Disclosure of the Invention

Problem to be Solved by the Invention

The present invention has been made to solve the problems under the above-mentioned circumstance and has as its object the provision of a method for the treatment of a crystal slurry, including a centrifugal separation step in which a slurry obtained by crystallizing a desired substance from a solution containing an organic component is subjected to solid liquid separation, the centrifugal separation step comprising carrying out centrifugal separation to form a cake, and then washing the formed cake with an organic solvent, so that the residual organic solvent content of the obtained crystals can be reduced to 1% by mass or less.

Means for Solving the Problem

The present inventors have made an earnest study with a view toward accomplishing the above object and, as a result, have found that the above object can be accomplished by a method for the treatment of a crystal slurry by centrifugal washing, comprising a centrifugal separation step in which a slurry obtained by crystallizing a desired substance from a solution containing an organic component is subjected to solid liquid separation, said step comprising first carrying out centrifugal separation to form a cake, and then washing the formed cake with an organic solvent. The present invention has been completed based on this funding.

That is, the present invention provides:

(1) A method for the treatment of a crystal slurry by centrifugal washing, comprising a centrifugal separation step in which a slurry obtained by crystallizing a desired substance from a solution containing an organic component is subjected to solid liquid separation, said step comprising first carrying out centrifugal separation to form a cake, and then washing the formed cake with an organic solvent to obtain crystals having a residual organic solvent content of 1% by mass or less;

(2) The method for the treatment of a crystal slurry as defined in above (1), wherein said centrifugal washing is a two stage process comprising a first stage in which the slurry is centrifuged to form the cake, and a second stage in which the formed cake is washed with the organic solvent;

(3) The method for the treatment of a crystal slurry as defined in above (1) or (2), wherein the organic solvent used for washing is an organic compound having a boiling point of 100°C or less;

(4) The method for the treatment of a crystal slurry as defined in any one of above (1) to (3), wherein the organic solvent is at least one organic solvent selected from the group consisting of methanol, ethanol, propanol, butanol, acetone, ethyl methyl ketone and petroleum hydrocarbon compounds having a boiling point of 55 to 100°C;

(5) The method for the treatment of a crystal slurry as defined in any one of above (1) to (4), wherein the residual organic solvent content of the treated crystals is 0.05 to 1% by mass; and

(6) The method for the treatment of a crystal slurry as defined in any one of above (1) to (5), wherein the crystal slurry is an adamantane slurry.

Effect of the Invention]

According to the present invention, it is possible to provide a method for the treatment of a crystal slurry, which includes a centrifugal separation step in which a slurry obtained by crystallizing a desired substance from a solution containing an organic component is subjected to solid liquid separation, the centrifugal separation step comprising carrying out centrifugal separation to form a cake, and then washing the formed cake with an organic solvent so that the residual organic solvent content of the obtained crystals can be reduced to 1% by mass or less.

Best Mode for Carrying Out the Invention

A method for the treatment of a crystal slurry by centrifugal washing according to the present invention is characterized in that, in its centrifugal separation step in which a slurry obtained by crystallizing a desired substance from a solution containing an organic component is subjected to solid liquid separation, a cake is first formed by centrifugation, and the formed cake is then washed with an organic solvent.

In the method for the treatment of a crystal slurry by centrifugal washing according to the present invention, the crystal obtained by crystallization is not specifically limited as long as it is an organic material crystal which has a hardness such that it is not crushed when subjected to a centrifugal force by a centrifugal separation device, which has a low porosity and which does not swell (or hardly swells) in organic solvents. A crystal of an adamantane may be mentioned as a preferred example of such a crystal. The particle size of the crystal is not specifically limited, either. Preferably, however, the crystal has an average particle diameter of 100 $\mu$m or more

A centrifugal washing device used for centrifugal washing of a crystal slurry in the present invention is not specifically limited as long as it has a structure permitting washing of a cake, obtained by centrifugal separation, with an organic solvent. Such a centrifugal washing device is not specifically limited as long as it has functions to first solid-liquid separate a slurry, obtained by crystallization, by centrifugal separation, to then spray an organic solvent over the fluid crystal cake, to wash off a mother liquor which deposits to surfaces of the crystals and a mother liquor and impurities which are included in aggregates of the crystals, to remove the solvent, and to discharge the crystal cake. A centrifugal washing device of a two stage type adapted for centrifuging the slurry to form a cake in the first stage and for washing the formed cake with an organic solvent in the second stage is also preferably used. Also preferably used is a horizontal type continuous centrifugal separation device which permits a continuous treatment and requires only a small installation space, which is high in solid-liquid separation efficiency and is excellent in treatment capacity and which has an inlet port for feeding an organic solvent for use in washing. Among them, a decanter of a rotary type provided inside with a screw conveyor or a decanter of a type which is composed of two unit structures (second unit is used for washing) each having a diameter reduced toward its crystal cake discharging side. As a preferred decanter, there may be mentioned a commercially available Model CR decanter manufactured by Tanabe Willtec Inc.

The organic solvent used for washing is preferably an organic compound having a boiling point of 100°C or less. Particularly preferred is an organic compound having a boiling point of about 30 to 100°C. More particularly, there may be mentioned at least one organic compound selected from the group consisting of methanol, ethanol, propanol, butanol, acetone, ethyl methyl ketone and petroleum hydrocarbon compounds having a boiling point of 55 to 100°C such as IPSOL-L (IP-L) (trade name, manufactured by Idemitsu Kosan Co., Ltd.).

The amount of the organic solvent used for washing is not specifically limited, but the organic solvent is generally used in an amount of about 0.5 to 10 liters per 1 kg of the crystal cake fed to the centrifugal washing device.

After the washing, the crystal cake is discharged with a high centrifugal force and at a high speed owing to the centrifugal separation machine. Therefore, the crystal cake is dispersed due to gas resistance to cause a great increase of the surface area. As a consequence, drying occurs instantaneously so that the residual content of the organic solvent in the crystal cake is reduced to 1% by mass or less, particularly about 0.05 to 1% by mass. Therefore, the crystals can be obtained in such a drainage state that substantially no further drying by a drying step is needed.

The residual content of the organic solvent in the crystal cake is measured as follows:

$$(W1 - W2)/W1 \times 100$$

where W1 represents a mass (g) of the crystal cake just discharged from the centrifugal separation machine, and W2 represents a mass (g) of the crystal cake after the just discharged crystal cake has been dried at a temperature of 40°C and a pressure of 10 kPa (absolute pressure) for 24 hours.

A portion of the separated mother liquor is generally discharged outside the system in order to prevent concentration of the impurities, with all or part of the remainder portion thereof being, if desired, recycled to the crystallization step.

Example

The present invention will be next described in more detail with adamantane as example. It should be noted, however, that the scope of the present invention is not limited to these examples in any way.

Example 1

(1) Catalyst Preparation Step:

In 2,000 kg of pure water were suspended, with stirring, 235 kg of sodium ion-exchanged Y-type zeolite (hereinafter referred to as NaY), to which a dilute aqueous nitric acid solution was added to adjust the pH of the suspended slurry to 5.5.

Then, a solution of 246 kg of lanthanum nitrate hexahydrate dissolved in 500 kg of warm water was gradually mixed into the above suspended slurry. The mixture was then heated to 90°C and stirred for 30 minutes, followed by filtration and washing. The washed cake was then dried overnight at 110°C and calcined at 600°C for 3 hours.

The obtained calcined powder was mixed again into 2,000 kg of pure water with stirring. The obtained suspended slurry was added with 228 kg of ammonium sulfate and stirred at 95°C for 30 minutes, followed by filtration and washing. The washed cake was again suspended in 2,000 kg of pure water and subjected to the similar ion exchanging treatment twice successively.

Thereafter, the formed cake was dried overnight at 110°C. This was placed in a tubular vessel and steamed at 510°C for 30 minutes with 100% steam. The obtained powder was then suspended in 2,000 kg of pure water, to which 32 kg of 25% by mass sulfuric acid were slowly added. Thereafter, the mixture was heated at 95°C for 30 minutes.

Subsequently, the mixture was filtered, washed and again suspended in 2,000 kg of pure water. The obtained suspension was added with 180 kg of a 1.71% by mass aqueous platinum tetramine chloride solution. The mixture was heated at 60°C for 30 minutes, filtered, washed, dried overnight at 110°C, thereby obtaining La-containing Y-type zeolite supporting 0.87% by mass of platinum by ion exchange.

(2) Reaction Step:

A stainless steel reaction tube having a total length of 3.5 m and a diameter of 24 cm was filled with 100 kg of the catalyst obtained in (1) above.

After the atmosphere had been substituted with nitrogen, hydrogen reduction was carried out at 300°C under ambient pressure for 2 hours in a hydrogen stream. Then, feed of trimethylene norbornane (TMN) (at a rate of 20 kg/hr), decalin as a dilution solvent (at a rate of 30 kg/hr) and hydrogen (at a rate providing a hydrogen/TMN molar ratio of 2.5) was started to continuously perform isomerization at 300°C under 5 MPa.

The reaction liquid was concentrated by atmospheric distillation (with 15 plates) at a tower bottom temperature of 180°C until an adamantane concentration of 30% by mass was reached.

(3) Refining Step:

The concentrate obtained in (2) above was used as a crystallization feedstock. Thus, 100 kg of the concentrate was charged in a dissolution and crystallization vessel and stirred at 120°C for dissolution. With continued stirring, the solution was cooled to 10°C to crystallize adamantane and to obtain a slurry of precipitated adamantane. The slurry was then fed to a horizontal-type centrifugal washing device at a feed rate of 244 kg/hr. Thereafter, IPSOL-L (IP-L, trade name, manufactured by Idemitsu Kosan Co., Ltd.) as an organic washing solvent was fed at a rate of 54 kg/hr to perform solid-liquid separation and washing. The obtained crystals had a residual solvent content of 0.1% by mass and were powdery crystals which did not substantially require to be dried.

Comparative Example 1

Solid-liquid separation and washing were performed in the same manner as that in Example 1 except that toluene was used as the organic washing solvent. The obtained crystals had a residual solvent content of 5% by mass. It was necessary to dry the crystals in a drying step.

Comparative Example 2

Solid-liquid separation was performed in the same manner as that in Example 1 except that a horizontal belt filter was used. The obtained crystals had a residual solvent content of 5 to 10% by mass. It was necessary to dry the crystals in a drying step.

Industrial Applicability

The present invention provides a method for the treatment of a crystal slurry, including a centrifugal separation step in which a slurry obtained by crystallizing a desired substance from a solution containing an organic component is subjected to solid liquid separation, the centrifugal separation step including carrying out centrifugal separation to form a cake, and then washing the formed cake with an organic solvent so that the residual organic solvent content of the obtained crystals can be reduced to 1% by mass or less.

**Claims**

1. A method for the treatment of a crystal slurry by centrifugal washing, comprising a centrifugal separation step in which a slurry obtained by crystallizing a desired substance from a solution containing an organic component is subjected to solid liquid separation, said step comprising first carrying out centrifugal separation to form a cake, and then washing the formed cake with an organic solvent to obtain crystals having a residual organic solvent content of 1% by mass or less.

2. The method for the treatment of a crystal slurry according to claim 1, wherein said centrifugal washing is a two stage process comprising a first stage in which the slurry is centrifuged to form the cake, and a second stage in which the formed cake is washed with the organic solvent.

3. The method for the treatment of a crystal slurry according to claim 1, wherein the organic solvent used for washing is an organic compound having a boiling point of 100°C or less.

4. The method for the treatment of a crystal slurry according to claim 1, wherein the organic solvent is at least one organic solvent selected from the group consisting of methanol, ethanol, propanol, butanol, acetone, ethyl methyl ketone and petroleum hydrocarbon compounds having a boiling point of 55 to 100°C.

5. The method for the treatment of a crystal slurry according to claim 1, wherein the residual organic solvent content of the treated crystals is 0.05 to 1% by mass.

6. The method for the treatment of a crystal slurry according to claim 1, wherein the crystal slurry is an adamantane slurry.

## INTERNATIONAL SEARCH REPORT

| International application No. |
| --- |
| PCT/JP2007/059573 |

A. CLASSIFICATION OF SUBJECT MATTER
*B01J19/00*(2006.01)i, *B01D9/02*(2006.01)i, *C07C7/14*(2006.01)i, *C07C13/615* (2006.01)i, *B01D21/26*(2006.01)n, *B04B1/20*(2006.01)n

According to International Patent Classification (IPC) or to both national classification and IPC

B. FIELDS SEARCHED

Minimum documentation searched (classification system followed by classification symbols)
C07C7/14, C07C13/615

Documentation searched other than minimum documentation to the extent that such documents are included in the fields searched
Jitsuyo Shinan Koho 1922-1996 Jitsuyo Shinan Toroku Koho 1996-2007
Kokai Jitsuyo Shinan Koho 1971-2007 Toroku Jitsuyo Shinan Koho 1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 61-76427 A (Nippon Steel Chemical Co., Ltd.),<br>18 April, 1986 (18.04.86),<br>Page 3, upper right column, line 11 to lower right column, line 8; table 1<br>(Family: none) | 1-5<br>6 |
| Y | JP 2004-59510 A (Idemitsu Petrochemical Co., Ltd.),<br>26 February, 2004 (26.02.04),<br>Par. Nos. [0011], [0012]<br>& WO 2004/011405 A1 & EP 1538139 A1<br>& TW 200403205 A & CN 1671636 A<br>& US 2006/111596 A1 & KR 2005078662 A | 6 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>01 June, 2007 (01.06.07) | Date of mailing of the international search report<br>12 June, 2007 (12.06.07) |
| --- | --- |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2007/059573 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 62-6527 B2  (Idemitsu Kosan Co., Ltd.),<br>12 February, 1987 (12.02.87),<br>Column 3, lines 10 to 15, column 4,<br>lines 15 to 26<br>(Family: none) | 6 |

Form PCT/ISA/210 (continuation of second sheet) (April 2005)

## INTERNATIONAL SEARCH REPORT

| International application No. |
| --- |
| PCT/JP2007/059573 |

**Box No. II    Observations where certain claims were found unsearchable (Continuation of item 2 of first sheet)**

This international search report has not been established in respect of certain claims under Article 17(2)(a) for the following reasons:

1. ☐  Claims Nos.:
because they relate to subject matter not required to be searched by this Authority, namely:

2. ☐  Claims Nos.:
because they relate to parts of the international application that do not comply with the prescribed requirements to such an extent that no meaningful international search can be carried out, specifically:

3. ☐  Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
        See extra sheet

1. ☐  As all required additional search fees were timely paid by the applicant, this international search report covers all searchable claims.

2. ☒  As all searchable claims could be searched without effort justifying an additional fee, this Authority did not invite payment of any additional fee.

3. ☐  As only some of the required additional search fees were timely paid by the applicant, this international search report covers only those claims for which fees were paid, specifically claims Nos.:

4. ☐  No required additional search fees were timely paid by the applicant.   Consequently, this international search report is restricted to the invention first mentioned in the claims; it is covered by claims Nos.:

**Remark on Protest**
the

☐  The additional search fees were accompanied by the applicant's protest and, where applicable, payment of a protest fee..

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet (2)) (April 2005)

| **INTERNATIONAL SEARCH REPORT** | International application No. |
|---|---|
| | PCT/JP2007/059573 |

Continuation of Box No.III of continuation of first sheet(2)

For the following reason, this international application involves three inventions which does not satisfy the requirement of unity of invention:
Main invention: "Claims 1-4"
Second invention: "Claim 5"
Third invention: "Claim 6"

As a result of search, the constitutions of claims 1-4 are disclosed in Document JP 61-76427 A (Nippon Steel Chemical Co., Ltd.), thus not being novel.
Therefore, the constitutions remain within the bounds of prior art and cannot be considered to be "special technical features" within the meaning of PCT Rule 13.2, second sentence.
It cannot be considered that there is a technical relationship between Main invention and Second or Third invention involving one or more of the same or corresponding special technical features.

Consequently, Main invention and Second or Third invention do not satisfy the requirement of unity of invention.

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004059510 A

- JP H0747209 B